# EUROPÄISCHE PATENTANMELDUNG

(11) **EP 2 111 871 A1**
(43) Veröffentlichungstag der Anmeldung: **28.10.2009**
(21) Anmeldenummer: 08008085.6
(22) Anmeldetag: 26.04.2008
(51) Int. Cl.: A61K 47/42, A61K 9/00

(54) **Stabilisierte Flüssigformulierung**

(71) Anmelder: Sandoz AG, 4056 Basel (CH)
(72) Erfinder: Matous, Heinrich Franz, A-6250 Kundl / Tirol (AT)
(74) Vertreter: Bohmann, Armin K.

(57) **Zusammenfassung**

Die vorliegende Erfindung umfasst eine Flüssigformulierung umfassend einen Wirkstoff und Protamin, wobei die Flüssigformulierung verschieden ist von einer Suspension.

## Beschreibung

Die vorliegende Erfindung betrifft Flüssigformulierungen umfassend einen Wirkstoff und Protamin, deren Verwendung und ein Verfahren zur Herstellung einer Flüssigformulierung.

Flüssigformulierungen stellen in der modernen Pharmazeutik wichtige Darreichunsgformen für pharmazeutisch aktive Wirkstoffe dar. Eine besonders relevante Untergruppe stellen die sogenannten Parenteralia dar, die zur Applikation in menschliches oder tierisches Gewebe durch Injektion oder Implantation bestimmt sind.

Obgleich nicht darauf beschränkt, enthalten Parenteralia typischerweise solche pharmazeutisch aktiven Verbindungen, die dem zu behandelnden Patienten direkt bzw. rasch zugeführt werden müssen, wie beispielsweise in der Notfallmedizin verwendete pharmazeutisch aktive Verbindungen, oder aber solche Verbindungen, die sich anderweitig nur schwer pharmazeutisch formulieren lassen. Dazu gehören bspw. pharmazeutisch aktive Verbindungen auf der Basis von Peptiden, Polypeptiden oder Proteinen.

Die Herstellung von parenteralen Zubereitungen erfordert eine Reihe von Hilfsstoffen, um beispielsweise die Isotonie zu gewährleisten, den pH-Wert einzustellen, die Löslichkeit zu verbessern, den Abbau der Wirkstoffe zu verhindern und um ausreichende antimikrobielle Eigenschaften zu erzielen. Diese Zusätze dürfen weder die medizinische Wirkung der Zubereitung beeinflussen, noch in der angewandten Konzentration lokal reizend oder sogar toxisch sein. Im Stand der Technik sind derartige Hilfsstoffe und Zusätze bekannt.

Peptide, Polypeptide und Proteine stellen vergleichsweise neue Wirkstoffklassen dar, die überwiegend mit der Entwicklung neuer, leistungsfähiger Syntheseverfahren bzw. biotechnologischer Verfahren in der erforderlichen Menge und Reinheit bereitgestellt werden können. Gerade die zunehmende therapeutische Verwendung von Polypeptiden und Proteinen wie bspw. von Wachstumsfaktoren, Cytokinen und Antikörpern erfordert Maßnahmen, um diese Wirkstoffe in der pharmazeutisch wirksamen Form bereitzustellen. Dazu gehört, ganz allgemein, die Stabilisierung der Polypeptide und Proteine, u.a. die Verhinderung der Aggregation derselben.

Im Bereich der Proteine wurde heuristisch die Verwendung von humanem Serumalbumin, polymeren Substanzen wie bspw. Polyethylenglykol, Dextranen oder Gelatine, Harnstoff oder einzelner Aminosäuren zur Stabilisierung von Flüssigformulierungen vorgenommen.

Der vorliegenden Erfindung liegt die Aufgabe zugrunde, eine Flüssigformulierung für einen pharmazeutisch aktiven Wirkstoff bereitzustellen. Des weiteren liegt der vorliegenden Anmeldung die Aufgabe zugrunde, eine klare Flüssigformulierung für einen pharmazeutisch aktiven Wirkstoff bereitzustellen. Darüber hinaus liegt der vorliegenden Erfindung die Aufgabe zugrunde, eine lagerstabile Flüssigformulierung für einen pharmazeutisch aktiven Wirkstoff bereitzustellen, wobei der pharmazeutisch aktive Wirkstoff bevorzugterweise aus der Gruppe ausgewählt ist, die Peptide, Polypeptide und Proteine umfasst. Schließlich liegt der vorliegenden Erfindung die Aufgabe zugrunde, ein Verfahren zur Herstellung einer Flüssigformulierung bereitzustellen.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Erfindungsgemäß wird die Aufgabe in einem ersten Aspekt gelöst durch eine Flüssigformulierung umfassend einen Wirkstoff und Protamin, wobei die Flüssigformulierung verschieden ist von einer Suspension.

In einer Ausführungsform ist vorgesehen, dass die Flüssigformulierung eine klare Flüssigformulierung ist.

In einer weiteren Ausführungsform ist vorgesehen, dass die Flüssigformulierung eine Trübung aufweist, die nicht stärker ist als die Trübung der Referenzlösung IV wie definiert in Ph.Eu 1997, Kapitel 2.2.1.

In einer noch weiteren Ausführungsform ist vorgesehen, dass das Protamin Lachsspermaprotamin ist.

In einer noch weiteren Ausführungsform ist vorgesehen, dass das Molverhältnis von Wirkstoff zu Protamin mindestens 1:2 oder mehr beträgt, bevorzugterweise mindestens 1:10 und bevorzugtererweise mindestens 1: 100.

In einer Ausführungsform ist vorgesehen, dass das Molverhältnis von Wirkstoff zu Protamin mindestens 1: 13 beträgt.

In einer Ausführungsform ist vorgesehen, dass der Wirkstoff ausgewählt ist aus der Gruppe umfassend Peptide, Polypeptide und Proteine.

In einer Ausführungsform ist vorgesehen, dass der Wirkstoff ein Molekulargewicht von etwa 3.000 Da bis etwa 200.000 Da, bevorzugterweise etwa 10.000 bis etwa 100.000 Da aufweist.

In einer Ausführungsform ist vorgesehen, dass der Wirkstoff ausgewählt ist aus der Gruppe, die Wachstumsfaktoren, Cytokine, Interferone, Interleukine und Antikörper umfasst.

In einer weiteren Ausführungsform ist vorgesehen, dass der Wirkstoff hGH ist.

In einer noch weiteren Ausführungsform ist vorgesehen, dass die Flüssigformulierung einen pH-Wert von etwa 4 bis 10, bevorzugterweise etwa 5,5 bis 7 aufweist.

In einer Ausführungsform ist vorgesehen, dass die Flüssigformulierung mindestens einen weiteren Hilfsstoff enthält, wobei der Hilfsstoff ausgewählt ist aus der Gruppe umfassend Glycin, Propylenglykol, Mannit und Poloxamer 188.

In einer Ausführungsform ist vorgesehen, dass die Flüssigformulierung zur Verwendung in einem Verfahren zur Behandlung einer Erkrankung vorgesehen ist.

In einem zweiten Aspekt wird die Aufgabe gelöst durch die Verwendung von Protamin zur Herstellung einer Flüssigformulierung.

In einer Ausführungsform des zweiten Aspekts ist vorgesehen, dass die Flüssigformulierung eine Flüssigformulierung nach dem ersten Aspekt ist.

In einem dritten Aspekt wird die Aufgabe gelöst durch die Verwendung von Protamin als Lösungsvermittler.

In einer Ausführungsform des dritten Aspekts ist vorgesehen, dass die Verwendung von Protamin im Rahmen der Herstellung einer Flüssigformulierung erfolgt, wobei bevorzugterweise die Flüssigformulierung eine Flüssigformulierung nach dem ersten Aspekt ist.

In einem vierten Aspekt wird die Aufgabe gelöst durch ein Verfahren zur Herstellung einer einen Wirkstoff und Protamin umfassenden Flüssigformulierung unfassend die Schritte:
a) Bereitstellen von Protamin;
b) Bereitstellen des Wirkstoffes; und
c) Formulieren von Protamin und des Wirkstoffes zu einer Flüssigformulierung

In einer Ausführungsform des vierten Aspektes ist vorgesehen, dass die Flüssigformulierung eine Flüssigformulierung nach dem ersten Aspekt ist.

Die vorliegenden Erfinder haben überraschend gefunden, dass Flüssigformulierungen von pharmazeutisch aktiven Verbindungen, insbesondere dann, wenn es sich um solche auf Peptid-, Polypeptid- oder Proteinbasis handelt, durch Zusatz von Protamin stabilisiert werden können bzw. die in der Flüssigformulierung enthaltene pharmazeutisch aktive Verbindung stabilisiert wird. Weiterhin haben die vorliegenden Erfinder überraschend festgestellt, dass der Zusatz von Protamin geeignet ist, die Kristallisation einer pharmazeutisch aktiven Verbindung zu verhindern, zumindest aber diese zu verringern. Dies gilt insbesondere dann, wenn die pharmazeutisch aktive Verbindung ein Peptid, Polypeptid oder Protein ist.

Die vorliegende Erfindung basiert des weiteren auf der Erkenntnis, dass Protamin als Lösungsvermittler im Zusammenhang mit Flüssigformulierungen und insbesondere klaren Flüssigformulierungen, wie sie hierin offenbart sind, verwendet werden kann.

Wie hierin verwendet bedeutet der Begriff, dass die Formulierung stabilisiert wird, in einer Ausführungsform, dass die Formulierung lagerstabil ist, bevorzugterweise die Protamin enthaltende Formulierung eine gegenüber der Formulierung ohne Protamin größere Lagerstabilität aufweist. Bevorzugterweise bedeutet eine größere Lagerstabilität, dass die Formulierung über einen längeren Zeitraum als ohne den Zusatz von Protamin gelagert werden kann, ohne dass dabei eine oder mehrere der Veränderungen auftreten, die die Lagerung oder Lagerungsfähigkeit beschränken. Veränderungen, die die Lagerung oder Lagerungsfähigkeit beschränken sind bspw. das Ausfallen oder die Kristallisation der pharmazeutisch aktiven Verbindung, eine Trübung oder eine Verfärbung der Flüssigstabilisierung. Diese Veränderungen können, abhängig von dem pharmazeutisch aktiven Agens verschiedene Ursachen haben, wie bspw. Komplexbildung, wobei der Komplex aus dem pharmazeutisch aktiven Wirkstoff oder aus dem pharmazeutisch aktiven Wirkstoff und einem oder mehreren der sonstigen Bestandteile der Formulierung bestehen kann, oder Abbau des pharmazeutisch aktiven Agens.

Es wird von den Fachleuten anerkannt werden, dass das hierin zu pharmazeutisch aktiven Verbindungen Gesagte für Wirkstoffe gleichermaßen gilt. Insoweit werden die Begriffe pharmazeutisch aktive Verbindung und Wirkstoff hierein in synonymer Weise verwendet, sofern nicht explizit Gegenteiliges angegeben ist.

Bei der erfindungsgemäßen Flüssigformulierung handelt es sich somit um eine lagerstabile Flüssigformulierung, die mindestens einen Wirkstoff und Protamin enthält.

Die erfindungsgemäße, mindestens einen Wirkstoff und Protamin enthaltende Flüssigformulierung ist verschieden von einer Suspension. Der Begriff der Suspension, wie hierin verwendet, bezeichnet insbesondere eine Arzneiform bestehend aus einem fließfähigen, grobdispersen Zwei-Phasensystem aus einer flüssigen äußeren und einer festen inneren Phase, wobei das System eine kontinuierliche äußere Phase in Form eines Dispersionsmittels aufweist, alleine unter dem Einfluss der Schwerkraft fließt, die Teilchengröße der dispersen Phase üblicherweise 1 - 100 µm und der Anteil der festen Phase 0,5 - 40 %, je nach Anwendungsbereich, beträgt.

Die erfindungsgemäße Formulierung ist insbesondere verschieden von einer Insulin-haltigen Flüssigformulierung, die Insulin enthält und unter dem Namen "Isophan" vermarktet wird, und einem Protamin enthaltenden FSME Vakzin, die als Suspensionen vorliegen. Die erfindungsgemäße Formulierung ist auch verschieden von einer Zubereitung von Protamin als Antidot für Heparin, wobei diese Zubereitung neben Protamin keine weitere pharmazeutisch aktive Verbindung und insbesondere keine weitere Substanz aus der Klasse der pharmazeutisch aktiven Verbindungen der Peptide, Polypeptide oder Proteine, enthält.

Demgegenüber stellt die erfindungsgemäße Flüssigformulierung eine klare Flüssigformulierung dar. Eine klare Flüssigformulierung im Sinne der vorliegenden Erfindung ist eine Flüssigformulierung, die eine Trübung aufweist, die nicht stärker ist als die Trübung der Referenzlösung IV, wie sie bevorzugterweise in dem Europäischen Arzneibuch, hierin auch abgekürzt als Ph.Eu., beschrieben ist, insbesondere in Kapitel 2.2.1 der Ph.Eu. 1997 "Klarheit und Opaleszenz von Flüssigkeiten". Die entsprechende Vorgehensweise ist hierin auch nochmals in Beispiel 1 beschrieben.

Protamin, wie bevorzugterweise hierin verwendet, bezeichnet eine Gruppe von Polypeptiden mit einem Molekulargewicht zwischen 1000 und 6000, die zu 80 - 85 % aus der Aminosäure L-Arginin bestehen, wobei der Rest der diese Polypeptide aufbauenden Aminosäuren L-Alanin, Glycin, L-Prolin, L-Serin, L-Isoleucin und L-Valin sind. Protamine sind stark alkalisch reagierende Verbindungen. Sie werden aus Vogel- oder Fischspermien durch Ausschütteln mit verdünnten Säuren gewonnen, wobei die stark basischen Protamine mit den Säuren Salze bilden. Je nach Herkunft unterscheidet man Clupein, das aus Heringen gewonnen wird, Cyprinin, das man aus Karpfen, Salmin aus Lachs und Iridin aus Forellen extrahiert. Protamine sind, bspw., in Ando, T., Yamasaki, M., Suzuki, K. "Protamines - Isolation, Characterization Structure and Function", Springer-Verlag Berlin Heidelberg New York, 1993, Seiten 1 - 114, beschrieben. In einer bevorzugten Ausführungsform der erfindungsgemäßen Flüssigformulierung handelt es sich bei dem Protamin um die freie Base von Lachsspermaprotamin, wie es bspw. von der Firma Sigma kommerziell erhältlich ist.

In der erfindungsgemäßen Formulierung ist das Protamin in einer Menge enthalten bzw. zu dieser zugegeben, so dass die Flüssigformulierung klar ist bzw. klar bleibt. Es ist im Rahmen der Fähigkeiten des Durchschnittsfachmannes im Lichte der vorliegenden Offenbarung, die Absolutmenge an Protamin ebenso wie das Molverhältnis von Protamin zu dem Wirkstoff durch einfache Routineverfahren zu bestimmen. Es wird von den Fachleuten anerkannt werden, dass die Absolutmenge an Protamin ebenso wie das Molverhältnis von Protamin zu dem Wirkstoff in der Flüssigformulierung von den speziellen Eigenschaften des Wirkstoffes abhängt. Eigenschaften des Wirkstoffes diesbezüglich sind die Größe und Konformation des Wirkstoffes sowie dessen Ladung und damit einhergehend dessen isoelektrischer Punkt.

Infolgedessen beträgt in bevorzugten Ausführungsformen das Molverhältnis von Wirkstoff zu Protamin mindestens 1: 2 oder mehr. Bevorzugtererweise beträgt das Molverhältnis mindestens 1:10 und noch bevorzugtererweise beträgt das Molverhältnis mindestens 1:100.

Hinsichtlich der in der erfindungsgemäßen Flüssigformulierung enthaltenen Absolutmenge an Protamin wird diese aufgrund der vorstehenden Beziehung im Rahmen von Routineuntersuchungen bestimmt. Dabei wird ausgehend von der in der Flüssigformulierung erwünschten bzw. enthaltenen Menge an Wirkstoff das Molverhältnis und damit auch die Absolutmenge an Protamin, wie sie in der konkreten Flüssigformulierung enthalten sein soll, bestimmt.

Der pH-Wert der erfindungsgemäßen Flüssigformulierung beträgt, u.a. auch in Anhängigkeit vom isoelektrischen Punkt des Wirkstoffes, etwa 4 bis 10, bevorzugterweise etwa 6 bis 7.

Die erfindungsgemäße Formulierung enthält bevorzugterweise als Wirkstoff ein oder mehrere Wirkstoffe, die ausgewählt sind aus der Gruppe umfassend Peptide, Polypeptide und Proteine. Es ist auch im Rahmen der vorliegenden Erfindung, dass der Wirkstoff verschieden ist von einem Peptid, Polypeptid oder einem Protein. In einer Ausführungsform ist vorgesehen, dass der Wirkstoff eine Kombination von mindestens zwei oder mehr Wirkstoffen ist. In einer bevorzugten Ausführungsform ist vorgesehen, dass mindestens einer der Wirkstoffe aus der Gruppe ausgewählt ist, die Peptide, Polypeptide und Proteine umfasst. In der Ausführungsform, bei der mindestens einer der Wirkstoffe aus der Gruppe ausgewählt ist, die Peptide, Polypeptide und Proteine umfasst, ist in einer Alternative vorgesehen, dass der mindestens ein weiterer Wirkstoff ebenfalls aus der Gruppe ausgewählt ist, die Peptide, Polypeptide und Proteine umfasst. In einer dazu alternativen Ausführungsform ist vorgesehen, dass der mindestens eine weitere Wirkstoff nicht aus der Gruppe ausgewählt ist, die Peptide, Polypeptide und Proteine umfasst. In einer noch weiteren Ausführungsform ist vorgesehen, dass die erfindungsgemäße Flüssigformulierung zwei oder mehr, bevorzugterweise drei, vier, fünf, sechs, sieben, acht, neun, zehn oder mehr Wirkstoffe enthält, wobei diese Wirkstoffe bevorzugterweise aus der Gruppe ausgewählt sind, die Peptide, Polypeptide und Proteins umfasst.

Es wird anerkannt werden, dass bei den Ausführungsformen, bei denen die erfindungsgemäße Flüssigformulierung neben Protamin mindestens zwei oder mehr Wirkstoffe enthält, die aus der Gruppe bestehend aus Peptiden, Polypeptiden und Proteinen einzeln und unabhängig voneinander ausgewählt sind, der Protamin-Gehalt der Flüssigformulierung gemäß der hierin gegebenen technischen Lehre ausgebildet werden muss. In diesem Falle ist bevorzugterweise ein jeder Wirkstoff einzeln zu betrachten, d.h. das erforderliche Molverhältnis eines jeden Wirkstoffes zu Protamin zu bestimmen bzw. festzulegen und anschließend die sich daraus für einen jeden einzelnen Wirkstoff ergebende Menge an Protamin der Flüssigformulierung zuzugeben. Der Gesamtgehalt an Protamin in der erfindungsgemäßen Flüssigformulierung ergibt sich in dieser Ausführungsform dann aus der Summe für jeden einzelnen Wirkstoff erforderlichen oder bestimmten Menge an Protamin.

In einer bevorzugten Ausführungsform wird hierin unter einem Peptid ein aus mehreren, durch Amidbindungen miteinander verbundenen Aminosäuren bestehendes Polymer bezeichnet, wobei die Anzahl der miteinander verknüpften Aminosäuren bevorzugterweise 50 nicht überschreitet. Es ist im Rahmen der vorliegenden Erfindung, dass Peptide aus L-Aminosäuren, D- Aminosäuren oder sowohl aus L- als auch aus D-Aminosäuren bestehen.

In einer bevorzugten Ausführungsform wird hierin unter einem Polypeptid ein Peptid verstanden, wie vorstehend beschrieben, welches aus mehr als 50 Aminosäuren besteht, bevorzugterweise aus 50 bis 100 Aminosäuren.

In einer bevorzugten Ausführungsform wird hierin unter einem Protein ein Peptid verstanden, wie vorstehend beschrieben, welches aus mehr als 100 Aminosäuren besteht. Es wird anerkannt werden, dass es im Stand der Technik andere als die oben angegebenen Definitionen für die Begrifflichkeiten existieren.

In einer weiteren Ausführungsform ist der in der erfindungsgemäßen Flüssigformulierung enthaltene Wirkstoff ein solcher mit einem Molekulargewicht von etwa 3000 Da bis etwa 200.000 Da, bevorzugterweise von etwa 10.000 Da bis etwa 100.000 Da.

In einer Ausführungsform ist der Wirkstoff ausgewählt aus der Gruppe umfassend Wachstumsfaktoren, Cytokine und Antikörper. Cytokine umfassen bevorzugterweise Interleukine, Interferone und Chemokine. Antikörper umfassen bevorzugterweise polyklonale Antikörper und monoklonale Antikörper sowie Fragmente davon. Bevorzugte Fragmente von Antikörpern sind Fab-Fragemente, F(ab)₂-Fragement und Fc-Fragmente.

In einer bevorzugten Ausführungsform ist der Wirkstoff humanes Wachstumshormon, das auch als hGH oder Somatropin bezeichnet wird.

Die Hauptindikationsgebiete von Somatropin sind der hypophysäre Minderwuchs von Kindern aufgrund einer ungenügenden Sekretion von körpereigenem Wachstumshormon, und Wachstumsstörungen von Mädchen die am Ullrich-Turner-Syndrom, einer X-Monosomie, leiden. Die Behandlung ist allerdings nur sinnvoll, wenn die Epiphysenfugen noch nicht geschlossen sind. Für den Erfolg der Therapie ist auch wichtig, dass damit rechzeitig begonnen wird und die Dosis laufend an den Bedarf des sich entwickelnden Kindes angepasst wird. Infolge der insulinähnlichen Wirkung und einer positiven Beeinflussung der Zellteilungsrate ist Somatropin bei Diabetes mellitus und Tumorerkrankungen kontraindiziert. Die Verabreichung erfolgt täglich über eine subkutane Injektion, wobei durch Applikation am Abend die beste Wirkung erzielt wird.

Somatropin besteht aus 191 Aminosäuren die untereinander durch zwei Disulfidbrücken verbunden sind (Cys53-Cys1165, Cys182-Cys189). In Lösung hat Somatropin Ähnlichkeit mit einem globulären Protein, die dreidimensionale Struktur enthält jedoch 4 ct-Helices die durch verlängerte ausgedehnte Ketten miteinander verbunden sind. Das Molekül enthält 3 Methionine, wovon 2 (Met14, Met125) empfindlich gegenüber Oxidation sind. Das Methionin in Position 170 ist wahrscheinlich aufgrund seiner Lage im Molekülinneren gegen Oxidationsprozesse abgeschirmt. Zudem enthält ein Molekül Somatropin 9 Asparagine und 13 Glutamine und somit viele reaktive Ansatzpunkte für eine Desaminierungsreaktion. Als Hauptangriffspunkt für die Desaminierung wird Asn 149 angegeben.

Die Desaminierung von Somatropin erfolgt über die Zwischenstufe eines cyclischen Imids, welches sich durch Hydrolyse in eine Iso-Aspartidsäure umlagert. Die Tendenz der Bildung des cyclischen Imids hängt von der Flexibilität der benachbarten C-terminalen Aminosäure ab. Das erklärt auch warum Asn 149 eine höhere Reaktivität aufweist als Asn 152. Diese Isomerisierung kann durch die Protein-Isoaspartyl-Methyltransferase-Reaktion nachgewiesen werden. Auch Asn 130 kommt als Isomerisierungsstelle im Molekül in Frage. Wie viele andere globuläre Proteine neigt auch Somatropin zur Ausbildung von Aggregaten, die entweder durch kovalente oder nicht-kovalente Bindungen entstehen können. Sowohl Schüttelprozesse, als auch das Gefrieren der wässrigen Lösung von Somatropin kann zur Bildung löslicher und unlöslicher Aggregate beitragen. Es sind diese unerwünschten Reaktionen, die mittels der erfindungsgemäßen Flüssigformulierung unterbunden oder zumindest verlangsamt werden.

In der Ausführungsform der erfindungsgemäßen Flüssigformulierung, bei der der Wirkstoff Somatropin ist, beträgt das Molverhältnis von Somatropin zu Protamin mindestens 1:13. Die Absolutmengen an Somatropin und Protamin entsprechen jenen, wie sie in den im Beispielteil angegeben Formulierungen genannt sind. Bevorzugterweise beträgt der pH-Wert dieser Flüssigformulierung zwischen 5,5 und 7, bevorzugterweise zwischen 5,8 und 6,5.

In einer Ausführungsform ist die erfindungsgemäße Flüssigformulierung eine wässrige Flüssigformulierung, die neben dem Wirkstoff und Protamin noch weitere, für Flüssigformulierungen und insbesondere Parenteralia übliche und den Fachleuten auf dem Gebiet bekannte weitere Bestandteile enthält. Zu den üblichen Bestandteilen gehören bspw. Puffer, Mittel zur Einstellung der Tonizität, Lösungsvermittler, Konservierungsmittel etc.

In einer Ausführungsform ist der Puffer ein Phosphat-Puffer auf der Grundlage von Natriumdihydrogenphosphat und Dinatriumhydrogenphosphat.

1,2-Propylenglykol kann als Konservierungsmittel, zur Einstellung der Tonizität oder auch als Lösungsvermittler verwendet werden. In Abhängigkeit von der beabsichtigen Verwendung variiert der Gehalt in der Flüssigformulierung und beträgt, bspw., 2,3 Gew.-%, wenn es als Mittel zur Einstellung der Isotonizität verwendet wird. Bevorzugterweise ist die Verbindung eine klare, farblose Flüssigkeit, die viskos und schwer flüchtig ist. Die Substanz ist geruchlos und hygroskopisch, hat einen Siedepunkt von 184-189° C und ist mit Wasser, niederen Alkoholen, Estern und Ketonen in jedem Verhältnis mischbar.

In einer Ausführungsform enthält die erfindungsgemäße Flüssigformulierung einen grenzflächenaktiven Lösungsvermittler, wobei der grenzflächenaktive Lösungsvermittler Poloxamer 188 ist. Poloxamere sind Polyole einer Serie von nahverwandten Block-CoPolymeren aus Ethylenoxid und Propylenoxid. Im Falle von Poloxamer 188 beträgt das durchschnittliche Molekulargewicht 7680 bis 9510, wobei der Anteil im Polymer von Ethylenoxid-Anteilen (a) 2 x 80, und der Anteil an Propylenoxid-Gruppen (b) 27 beträgt.

Mannit ist ein Beispiel für einen physiologisch indifferenten Hilfsstoff, der als Gerüstbildner, aber auch als Stabilisator wirkt, wobei die Stabilisierung wohl über die OH-Gruppen erfolgt. Der Gehalt an Mannit in pharmazeutischen Formulierungen kann bis zu 50 Gew.-% betragen.

Glycin wird zur Einstellung des pH- Wertes, aber auch der Tonizität verwendet. Typische Konzentrationen von Glycin in pharmazeutischen Formulierungen betragen bis zu 90 Gew. %. In einer bevorzugten Ausführungsform enthält die Flüssigformulierung Glycin.

Im Bereich der Flüssigformulierungen werden sogenannte Hilfsstofflösungen verwendet, die die Grundlage für die Flüssigformulierung bilden und zu der insbesondere der Wirkstoff und ggf. weitere Bestandteil der Flüssigformulierung hinzugegeben werden. Beispielhafte Hilfsstofflösungen sind die in Beispiel 2 angegebene Mannit-Formulierung, die Glycin-Formulierung und Glycin-Propylenglykol-Formulierung. Es ist im Rahmen der vorliegenden Erfindung, dass diese Hilfsstofflösungen auch im Zusammenhang mit anderen Wirkstoffen als dem in den Beispielen angegebenen Somatropin verwendet werden können.

In einer Ausführungsform der erfindungsgemäßen Flüssigformulierung ist vorgesehen, dass es sich um eine pharmazeutische Flüssigformulierung handelt.

In einer weiteren Ausführungsform ist vorgesehen, dass die erfindungsgemäße Flüssigformulierung bei der Behandlung einer Erkrankung bzw. zur Herstellung eines Medikamentes zur Behandlung einer Erkrankung verwendet wird. Es wird von den Fachleuten anerkannt werden, dass die Erkrankung, die unter Verwendung der erfindungsgemäßen Flüssigformulierung behandelt werden kann, durch den in der Flüssigformulierung enthaltenen Wirkstoff bedingt wird. Wie hierin verwendet umfasst der Begriff der Behandlung auch die Prävention der entsprechenden Erkrankung.

Schließlich betrifft die vorliegende Erfindung ein Verfahren zur Herstellung einer einen Wirkstoff und Protamin umfassenden Flüssigformulierung umfassend die Schritte:
a) Bereitstellen von Protamin;
b) Bereitstellen des Wirkstoffes bzw. der Wirkstoffe; und
c) Formulieren von Protamin und des Wirkstoffes zu einer Flüssigformulierung.

Im Prinzip kann das Verfahren gemäß üblicher und den Fachleuten auf dem Gebiet bekannten Vorgehens- und Verfahrensweisen durchgeführt werden. In einer Ausführungsform wird das Protamin in einer Hilfsstofflösung, insbesondere einer solchen, wie sie hierin beschrieben ist, bereitgestellt und dann der Wirkstoff zugegeben. Es ist jedoch auch im Rahmen der vorliegenden Erfindung, zuerst den Wirkstoff zu der Hilfsstofflösung zuzugeben und dann Protamin zuzugeben. In einer Ausführungsform ist vorgesehen, dass der pH-Wert der Hilfsstofflösung so eingestellt wird, dass sowohl der isolelektrische Punkt von Protamin als auch der des Wirkstoffes bzw. ein Durchschreiten des/der isoelektrischen Punkte(s) bei Zugabe von Protamin bzw. dem Wirkstoff vermieden wird. Schließlich ist in einer Ausführungsform vorgesehen, dass Glycin der Flüssigformulierung zugegeben wird, bevorzugterweise in einer Hilfsstofflösung.

### Beispiel 1: Verfahren zur Bestimmung der Trübung von Flüssigformulierungen

Das nachfolgend beschriebene Verfahren zur Bestimmung der Trübung von Flüssigformulierungen entspricht dem in Ph.Eu. 1997, Kapitel 2.2.1 beschrieben Verfahren.

In identischen Neßler-Zylindern aus farblosem, durchsichtigem Neutralglas mit einem inneren Durchmesser von 15 bis 25 mm und mit flachem Boden wird die zu prüfende Flüssigkeit mit der unten beschriebenen, frisch hergestellten Referenzsuspension in einer Schichtdicke von 40 mm verglichen. 5 min nach der Herstellung der Referenzsuspension werden die Flüssigkeiten in vertikaler Durchsicht gegen einen dunklen Untergrund und bei diffusem Tageslicht geprüft.

Die Lichtverteilung muß so sein, daß die Referenzsuspension I von Wasser R und die Referenzsuspension II von der Referenzsuspension I leicht zu unterscheiden ist.

Eine Flüssigkeit wird als *klar* bezeichnet, wenn die Klarheit unter den oben angegebenen Bedingungen derjenigen von Wasser R oder des verwendeten Lösungsmittels entspricht oder wenn die Flüssigkeit nicht stärker opalesziert als die Referenzsuspension I.

### Reagenzien

*Hydrazinsulfat-Lösung:* 1,0 g Hydrazinsulfat R wird in Wasser R zu 100,0 ml gelöst. Die Lösung wird 4 bis 6 h lang stehengelassen.

*Methenamin-Lösung:* 2,5 g Methenamin R werden in 25,0 ml Wasser R in einem 100-ml-Erlenmeyerkolben mit Schliffstopfen gelöst.

*Opaleszenz-Stammsuspension:* 25,0 ml der Hydrazinsulfat-Lösung werden in den Erlenmeyerkolben mit der Methenamin-Lösung gebracht, gemischt und 24 h lang stehengelassen. Diese Suspension kann 2 Monate lang in einem Glasbehältnis mit intakter Oberfläche aufbewahrt werden. Die Suspension darf nicht an der Wand des Behältnisses kleben und muß vor Verwendung sorgfältig geschüttelt werden.

*Opaleszenz-Referenzsuspension:* 15,0 ml Opaleszenz-Stammsuspension werden mit Wasser R zu 1000,0 ml verdünnt. Die Suspension ist bei Bedarf frisch herzustellen und darf höchstens 24 h lang verwendet werden.

*Referenzsuspensionen:* Die Referenzsuspensionen werden nach folgender Tabelle hergestellt. Sie werden unmittelbar vor der Verwendung hergestellt und umgeschüttelt.

| | **I** | **II** | **III** | **IV** |
|---|---|---|---|---|
| Opaleszenz Referenzsuspension | 5,0 ml | 10,0 ml | 30,0 ml | 50,0 ml |
| Wasser *R* | 95,0 ml | 90,0 ml | 70,0 ml | 50,0 ml |

### Beispiel 2: Hilfsstoffformulierungen

Die folgenden Hilfsstoffformulierungen können im Rahmen der vorliegenden Erfindung verwendet werden.

Rezeptur der Somatropin enthaltenden Flüssigformulierung basierend auf der Mannit enthaltenden Hilfsstoffformulierung. Die Hilfsstoffformulierung entspricht der nachfolgenden Rezeptur ohne den Wirkstoff Somatropin.

| ***Bestandteile*** | ***Molekulargewicht [g*/*mol]*** | ***mg*/*ml Formalierung*** |
|---|---|---|
| **Wirkstoff** | | |
| Somatropin | 22129 | 3,33 mg/ml (151 µM) |

| **Hilfsstoffe** | | |
|---|---|---|
| Na₂HPO₄ x 7H₂O | 268,07 | 0,88 mg/ml (3,2 mM) |
| NaH₂PO₄x2H₂O | 156,01 | 1,039 mg/ml (6,7 mM) |
| Mannit | 182,18 | 35,11 mg/ml (192,7 mM) |
| Poloxamer 188 | 8595 | 1,98 mg/ml (231 µM) |
| Benzylalkohol | 108,14 | 8,93 mg/ml (82,6 mM) |
| Wasser | 18,02 | ad 12,26 ml |
| H₃PO₄ | 98,00 | ad pH= 6,2 |
| Protamin | 4100 | 82mg/ml- 0,001 µg/ml |

Rezeptur der Somatropin enthaltenden Flüssigformulierung basierend auf der Glycin enthaltenden Hilfsstoffformulierung. Die Hilfsstoffformulierung entspricht der nachfolgenden Rezeptur ohne den Wirkstoff Somatropin.

| ***Bestandteile*** | ***Molekulargewicht [g*/*mol]*** | ***mg*/*ml Formulierung*** |
|---|---|---|
| **Wirkstoff** | | |
| Somatropin | 22129 | 3,33 mg/ml (151 µM) |

| **Hilfsstoffe** | | |
|---|---|---|
| Na₂HPO₄ x 7H₂O | 268,07 | 0,88 mg/ml (3,2 mM) |
| NaH₂PO₄x2H₂O | 156,01 | 1,039 mg/ml (6,7 mM) |
| Glycin | 75,07 | 7,43 mg/ml (98,9 mM) |
| Poloxamer 188 | 8595 | 1,98 mg/ml (231 µM) |
| Benzylalkohol | 108,14 | 8,93 mg/ml (82,6 mM) |
| Wasser | 18,02 | ad 12,26 ml |
| H₃PO₄ | 98,00 | ad pH= 6,2 |
| Protamin | 4100 | 75 mg/ml- 0,001 µg/ml |

Rezeptur der Somatropin enthaltenden Flüssigformulierung basierend auf der Glycin-Propylenglykol enthaltenden Hilfsstoffformulierung. Die Hilfsstoffformulierung entspricht der nachfolgenden Rezeptur ohne den Wirkstoff Somatropin.

| ***Bestandteile*** | ***Molekulargewicht [g*/*mol]*** | ***mg*/*ml Formulierung*** |
|---|---|---|
| **Wirkstoff** | | |
| Somatropin | 22129 | 3,33 mg/ml (151 µM) |

| **Hilfsstoffe** | | |
|---|---|---|
| Na₂HPO₄ x 7H₂O | 268,07 | 0,88 mg/ml (3,2 mM) |
| NaH₂PO₄x2H₂O | 156,01 | 1,039 mg/ml (6,7 mM) |
| Glycin | 75,07 | 7,43 mg/ml (98,9 mM) |
| Propylenglykol | 76,10 | 7,43 mg/ml (97,6 mM) |
| Poloxamer 188 | 8595 | 1,98 mg/ml (231 µM) |
| Benzylalkohol | 108,14 | 8,93 mg/ml (82,6 mM) |
| Wasser | 18,02 | ad 12,26 ml |
| H₃PO₄ | 98,00 | ad pH= 6,2 |
| Protamin | 4100 | 82 mg/ml- 0,02 µg/ml |

### Beispiel 3: Herstellung verschiedener Protamin-haltiger Flüssigformulierungen

Bei der Herstellung der verschiedenen Flüssigformulierungen wurde von einer Somatropin enthaltenden Stammlösung ausgegangen, die auch als Bulk Solution bezeichnet wird. Die Bulk Solution enthält das rekombinant hergestellte Somatropin zu 10, 1 mg/ml, wie durch Größenausschlußchromatographie bestimmt.

Die Herstellung der Ansätze erfolgte im kleinen Maßstab. Da es vor jedem Versuch nötig war, die Bulk Solution schonend von -20 °C auf Raumtemperatur aufzutauen, wurde die jeweilige Versuchsgröße auf die verfügbaren Portionen der Wirkstofflösung abgestimmt, um ein erneutes Einfrieren und Auftauen des empfindlichen Proteins zu verhindern.

Um die Sterilität während des Herstellungsprozesses zu gewährleisten, wurden die Formulierungen aseptisch unter der Laminar-Flow-Box hergestellt. Zudem wurde darauf geachtet, jede der Komponenten vor ihrer Anwendung durch einen Spritzenvorsatzfilter (Acrodisc Syringe Filter, Fa. Gelman Laboratory) mit einem definierten Porendurchmesser von 0,22 µm zu filtrieren.

Im folgenden sowie in Beispiel 4 werden die verschiedenen Protamin-haltigen Flüssigformulierungen auch als "Versuche" bezeichnet. Grundlage für zumindest einige der Versuche war die als EP200AQ-Formulierung bezeichnete Formulierung, die die folgende Zusammensetzung aufweist:

| ***Bestandteile*** | ***Molekulargewicht [g*/*mol]*** | ***mg*/*ml Formulierung*** |
|---|---|---|
| **Wirkstoff** | | |
| Somatropin | 22129 | 3,33 mg/ml (151 µM) |

| **Hilfsstoffe** | | |
|---|---|---|
| Na₂HPO₄ x 7H₂O | 268,07 | 0,88 mg/ml (3,2 mM) |
| NaH₂PO₄ x 2H₂O | 156,01 | 1,039 mg/ml (6,7 mM) |
| Mannit | 182,18 | 35,11 mg/ml (192,7 mM) |
| Poloxamer 188 | 8595 | 1,98 mg/ml (231 µM) |
| Benzylalkohol | 108,14 | 8,93 mg/ml (82,6 mM) |
| Wasser | 18,02 | ad 12,26 ml |
| H₃PO₄ | 98,00 | ad pH= 6,2 |

### Herstellungsvorgang der Versuche 1 und 2

- 2090 µl Bulk Solution (pH-Wert 7,3) werden vorgelegt
- 910 µl wässrige Protaminlösung (0,84 mg Protamin/ml Formulierung) werden langsam zugegeben, dies entspricht 90% des Wassers des gesamten Versuches (stark basisch, pH-Wert steigt mit zunehmender Protaminkonzentration)
- anschließend werden 3040 µl Hilfsstofflösung zudosiert (pH-Wert 3,80)
- bei der so erhaltenen Lösung wird mit 1 M H₃PO₄ ein pH-Wert von 6,2 eingestellt
- Auffüllung mit Aqua bidest auf das geforderte Endvolumen

### Versuch 1

Bei diesem Versuch wurde eine wirkstofffreie EP2000AQ-Formulierung hergestellt. Zur Pufferlösung, die in diesem Fall nur aus Phosphaten und Aqua bidest besteht, wurde die wässrige Protaminlösung, die 90% des Gesamtwassers der Formulierung enthält, beigefügt, anschließend die Hilfsstofflösung zudosiert, mit 1 M H₃PO₄ ein pH- Wert von 6,2 eingestellt und mit Wasser auf das geforderte Endgewicht aufgefüllt.

### Versuch 2

Bei diesem Versuch entstand eine EP2000AQ-Formulierung mit einem Molverhältnis hGH zu Protamin von 6:1 (0,84 mg/ml Formulierung). Die Vereinigung der Komponenten erfolgte analog zu Versuch 1.

Herstellungsvorgang der Ansätze 3, 4, 5, 6, 7
- 2090 µl Bulk Solution (pH-Wert 7,3) werden vorgelegt
- 505 µl Wasser zugegeben, dies entspricht 50% des Wassers des gesamten Versuches
- anschließend werden 3040 µl Hilfsstofflösung zudosiert (pH-Wert 3,80)
- an dieser Stelle wird mit 404 µl der wässrigen Protaminlösung versetzt
- bei der so erhaltenen Lösung mit 1 M H₃PO₄ ein pH-Wert von 6,2 eingestellt
- Auffüllung mit Aqua bidest auf ein Endvolumen von 6130 µl

### Versuch 3

Im Zuge dieses Versuches wurde eine EP 2000AQ-Formulierung mit molaren hGH-Protamin-Verhältnissen von 1:0,02 (0,4 µg Protamin/ml Formulierung) und 1:0,002 (0,04 µg Protamin/ml Formulierung) hergestellt. Der Herstellungsprozess wurde insofern abgeändert, als man die Protaminlösung nicht unmittelbar zur Bulk Solution zudosierte, sondern durch die Zugabe von Wasser und Hilfsstofflösung eine Art "Pufferzone" zwischen den beiden Proteinen schuf.

### Versuch 4

Auch bei diesem Versuch wurde die Protaminlösung nach dem Beifügen der Hilfsstofflösung zudosiert. Das molare Verhältnis zwischen den zwei Proteinen beträgt hGH zu Protamin 1:0,04 (0,08 µg Protamin/ml Formulierung).

### Versuch 5

Die Herstellung der Formulierung ist in Anlehnung an die Ansätze 3 und 4 erfolgt. Das Molverhältnis zwischen hGH und Protamin wurde von 1:0,04 auf 1:0,1 (2 µg Protamin/ml Formulierung) bzw. 1:0,2 (4 µg Protamin/ml Formulierung) abgeändert.

### Versuch 6

Bei diesem Versuch wurde der Herstellungsvorgang beibehalten. Die molaren Verhältnisse reichen von 1:0,1 (2 µg Protamin/ml Formulierung) über 1:0,08 (1,6 µg Protamin/ml Formulierung) 1:0,06 (1,2 µg Protamin/ml Formulierung), 1:0,04 (0,8 µg Protamin/ml Formulierung) bis 1:0,02 (0,4 µg Protamin/ml Formulierung). Die wässrigen Protaminlösungen ergaben sich durch Verdünnung einer Protamin-Stammlösung mit Aqua bidest.

Versuch 7

Im Zuge dieses Versuches wurde die Mannit-Hilfsstofflösung durch eine Glycin/Propylenglykol-Hilfsstofflösung ersetzt (Rezeptur siehe Tabelle 10). Die Protaminlösungen wurden wiederum durch Verdünnung einer Protamin-Stammlösung mit Aqua bidest hergestellt. Wie in Versuch 6 wurden auch in diesem Fall Formulierungen mit Molverhältnissen hGH zu Protamin von 1:0,1 (2 µg Protamin/ml Formulierung) bis 1:0,02 (0,4 µg Protamin/ml Formulierung) hergestellt.

### Versuche 8, 9, 10

In Anlehnung an die bisherige Formulierungsarbeit war die nächste Überlegung, inwieweit jeder einzelne Hilfsstoff Einfluss auf mögliche Interaktionen zwischen hGH und Protamin hat. Zu diesem Zweck wurden parallel Mannit- und Glycin/Propylenglykol-Formulierungen hergestellt, wobei pro Versuch jeweils eine Komponente der Hilfsstofflösung entfernt wurde. Angefangen von Benzylalkohol über Poloxamer 188, Propylenglykol und Mannit wurde kontinuierlich die Anzahl der Komponenten reduziert, sodass letztlich ein Versuch mit Pufferlösung und Glycin bzw. einer der nur aus Pufferlösung besteht zur Verfügung stand. Unmittelbar nach der Herstellung wurden die Endformulierungen mittels pH-Meter vermessen.

### Versuch 11

Um die Reproduzierbarkeit der Ergebnisse zu gewährleisten, wurde eine weitere Serie mit Mannit-Hilfsstofflösung bzw. Glycin/Propylenglykol-Hilfsstofflösung hergestellt. Am Herstellungsprozess wurde nichts verändert, die molaren Verhältnisse von hGH und Protamin waren wie folgt 1:0,2 (4 µg Protamin/ml Formulierung); 1:0,1 (2 µg Protamin/ml Formulierung); 1:0,08 (1,6 µg Protamin/ml Formulierung); 1:0,06 (1,2 µg Protamin/ml Formulierung); 1:0,04 (0,8 µg Protamin/ml Formulierung) und 1:0,02 (0,4 µg Protamin/ml Formulierung).

Um beim Sterilfiltrieren sicher gehen zu können, dass der Filter mit Protamin abgesättigt wird, wurde für die Protamin-Stammlösung ein größerer Ansatz berechnet und der Filter zuvor mit zirka 3 ml Protaminlösung gespült. Auch in diesem Fall wurde unmittelbar nach der Herstellung der pH-Wert der Endformulierungen bestimmt.

Herstellungsvorgang der Versuche 12, 13, 14, 15
- 2090 µl Bulk Solution (pH-Wert 7,3) werden vorgelegt
- 1010 µl Wasser werden zugegeben, dies entspricht 50% des Wassers des gesamten Versuches
- anschließend werden 6080 µl Hilfsstofflösung zudosiert (pH-Wert 3,80)
- an dieser Stelle wird mit 808 µl der wässrigen Protaminlösung versetzt
- bei der so erhaltenen Lösung wird mit 1 M H₃PO₄ bzw. 0,1 M H₃PO₄ ein pH-Wert von 6,2 eingestellt
- Ergänzung mit Aqua bidest auf ein Endvolumen von 6130 µl

### Versuch 12

Aufgrund der bisherigen Ergebnisse hat man sich dafür entschieden die Mannit-Formulierung vorerst außer acht zu lassen, und konzentrierte sich auf die Glycin/Propylenglykol- bzw. die Glycin-Hilfsstofflösung. Weiteres wurde beschlossen den Versuch zu verdoppeln, was auch die Herstellung der Protamin-Stammlösung erleichterte.

hGH und Protamin wurde in den Molverhältnissen 1:0,5 (10 µg Protamin/ml Formulierung); 1:0,2 (4 µg Protamin/ml Formulierung); 1:0,1 (2 µg Protamin/ml Formulierung); 1:0,05 (1 µg Protamin/ml Formulierung); 1:0,02 (0,4 µg Protamin/ml Formulierung) und 1:0;01 (0,2 µg Protamin/ml Formulierung) eingesetzt.

Zusätzlich wurde, um die pH-Wert-Korrektur zu erleichtern, alternativ zu einer 1M H₃PO₄ eine 0,1 M H₃PO₄ verwendet. Auch bei diesem Versuch wurden im Anschluss an die Herstellung die Endformulierungen im pH-Meter vermessen und anschließend auf einen Wert von 5,8 korrigiert. Um die Proben auf Stabilität legen zu können, wurde letztlich mit 0,5 M Natronlauge ein pH-Wert von 6,2 eingestellt.

Versuch 13

Es kamen zwei verschiedenen Hilfsstofflösungen zum Einsatz. Hilfsstofflösung I enthält Glycin und Propylenglykol, Hilfsstofflösung II enthält Mannit.

Dieser Versuch unterscheidet sich insofern von den vorherigen, als der pH-Wert der Formulierung nach Zugabe von Bulk Solution, Wasser und Hilfsstofflösung mit 0,5 N Natronlauge auf einen Wert von 7 eingestellt wurde, wobei zuerst der pH-Wert dieser Mischung aus Bulk Solution, Wasser und Hilfsstofflösung an sich bestimmt wurde. Die verwendeten Molverhältnisse waren wie folgt hGH zu Protamin 1:0,05 (1 µg Protamin/ml Formulierung); 1:0,02 (0,4 µg Protamin/ml Formulierung) und 1:0,01(0,2 µg Protamin/ml Formulierung).

Nach Korrektur auf einen pH-Wert von 7, zudosieren der wässrigen Protaminlösung und des restlichen Wassers, kam es zur Bestimmung des pH-Wertes der Endformulierung, und zum Austesten wie weit er gesenkt werden kann bis hGH seinen IP erreicht und ausfällt.

### Versuch 14

Zur Anwendung kam die Mannit-Hilfsstofflösung. Das hGH-Protamin-Verhältnis betrug wiederum 1:0,05 (1 µg Protamin/ml Formulierung); 1:0,02 (0,4 µg Protamin/ml Formulierung) und 1:0,01(0,2 µg Protamin/ml Formulierung).

Im Unterschied zu Versuch 13 erfolgte hier die Bestimmung des pH-Wertes der Lösungen erst nach Fertigstellung der Formulierungen. Im Anschluss daran wurde wiederum ausgetestet wie weit der pH-Wert gesenkt werden kann, bis es zum Ausfällen von hGH kommt.

### Versuch 15

Im Gegensatz zu vorhergehenden Versuchen wurde in dieser Testreihe sowohl im Mannit- als auch im Glycin/Propylenglykol-Versuch die Protaminkonzentration gesenkt. Die Formulierungen enthalten hGH und Protamin in Molverhältnissen von 1:0,005 (0,1 µg Protamin/ml Formulierung) und 1:0,001 (0,02 µg Protamin/ml Formulierung). Der pH-Wert der Formulierung wurde nach Mischung von Bulk Solution, Wasser und Hilfsstofflösung mit 0,5 N Natronlauge auf einen Wert von 7 eingestellt. Anschließend wurde die Protaminlösung hinzugefügt, mit Wasser auf ein Zielvolumen von 12260 µl aufgefüllt und der pH-Wert auf einen Wert von ungefähr 5 eingestellt.

Herstellungsvorgang der Versuche 16, 17, 18, 19
- pH-Wert-Korrektur der Hilfsstofflösung auf einen geforderten Wert
- Einführen von Protamin als zusätzliche Komponente der Hilfsstofflösungen; Protamin wird direkt in der Hilfsstofflösung gelöst
- Verdünnung der Stamm-Hilfsstofflösung mit weiterer Hilfsstofflösung um die gewünschte Protaminkonzentration zu erreichen
- Vorsichtiges Zudosieren der Bulk Solution
- Einstellung des Ziel-pH-Wertes der Endformulierung
- Abzentrifugieren eventuell auftretender Niederschläge
- Abfüllung in 2R-Vials und Lagerung bei Raumtemperatur bzw. bei 2-8° C

### Versuch 16

In diesem Versuch wurde das Protamin sowohl für den Mannit- als auch für den Glycin/Propylenglykol-Versuch in den Hilfsstofflösungen gelöst, anschließend die Bulk Solution zudosiert und der pH-Wert ermittelt. Vor dem Lösen des Protamins der Hilfsstofflösung, wurde bei dieser ein pH-Wert von 7 eingestellt. In 30 µl-Schritten wurden unter ständigem Umschütteln 330 µl der Bulk Solution zugesetzt. Das Molverhältnis hGH zu Protamin beträgt 1:267 (75 mg Protamin/ml Formulierung). Im Anschluss wurde der pH-Wert mit 1M H₃PO₄ auf einen Ziel-pH-Wert von 6,00 gesenkt.

### Versuch 17

Dieser Versuch unterscheidet sich von Versuch 16 dadurch, dass die Hilfsstofflösungen in diesem Fall anstatt eines pH-Wertes von 7,0 einen von 3,6 aufweisen. Das Molverhältnis hGH zu Protamin beträgt wiederum 1:267 (75 mg Protamin/ml Formulierung).

Auch in diesem Fall wurde der pH-Wert auf einen Ziel-pH-Wert von 6,00 korrigiert.

Versuche 18,19

Da sich die Abänderung des Herstellungsprozesses bewährt hat, wurde die Versuchgröße wesentlich erhöht um für nachfolgende Analytik genügend Muster auf Stabilität legen zu können. Zum Einsatz kamen sowohl die Mannit- als auch die Glycin-Hilfsstofflösung. Nach Lösen des Protamins in den Hilfsstofflösungen wurde jeweils ein pH-Wert von 7 eingestellt. Es wurden jeweils vier hGH-Protamin-Molverhältnisse hergestellt und diese auf vier verschiedene pH-Werte korrigiert. Bei den verwendeten Molverhältnissen handelte es sich um hGH zu Protamin 1: 100 (53,5 mg Protamin/ml Formulierung); 1:10 (5,35 mg Protamin/ml Formulierung); 1:1 (0,53 mg Protamin/ml Formulierung) und 1:0,1 (0,05 mg Protamin/ml Formulierung).

Die mit 1 M H₃PO₄, 0,1 M H₃PO₄ und 0,1 M Natronlauge eingestellten pH-Werte reichten von 5,8 über 6,0 und 6,2 bis zu einem Wert von 6,5.

### Beispiel 4: Stabilität der Protamin-haltigen Flüssigformulierungen

Die Ergebnisse zur Stabilität der in Beispiel 3 beschriebenen Protamin-haltigen Flüssigformulierungen und insbesondere des Einflusses von Protamin auf die Stabilität, gemessen auf der Grundlage der Trübung der Flüssigformulierung, sind in diesem Beispiel zusammengefasst.

Die vorstehenden Ergebnisse hinsichtlich Stabilität der Stabilität von Somatropin in Protaminenthaltenden Flüssigformulierungen wurden auch durch CD-Spektroskopie bestätigt.

### Versuch 1

Nach Zugabe aller Komponenten wies die wirkstofffreie Formulierung einen pH-Wert von 10 auf, und konnte mit 1 M Phosphorsäure problemlos auf einen Wert von 6,2 korrigiert werden.

| Versuch | Molverhältnis hGH: Protamin | Komp. 1 | Komp. 2 | Komp.3 | Komp.4 | Komp. 5 | Komp. 6 |
|---|---|---|---|---|---|---|---|
| 1 | | | Wässrige Protaminlösung | Hilfsstofflösung (Mannit) | pH-Wert-Einstellung auf 6,2 | Aqua bidest | |
| 2 | 6:1 | Bulk Solution | Wässrige Protaminlösung | Hilfsstofflösung (Mannit) | pH-Wert Einstellung auf 6,2 | Aqua bidest | |
| 3 | 1:0,02 1:0002 | Bulk Solution | Aqua bidest (50% des Gesamt wassers) | Hilfsstofflösung (Mannit) | Wässrige Protaminlösung | pH-Wert-Einstellung auf 6,2 auf 6,2 | Mit Aqua bidest auf Endgewicht Auffüllen |
| 4 | 1:0,04 | Bulk Solution | Aqua bidest (50% des Gesamt wassers) | Hilfsstofflösung (Mannit) | Wässrige Protaminlösung | pH-Wert-Einstellung auf 6,2 | Mit Aqua bidest auf Endgewicht Auffüllen |
| 5 | 1:0,1; 1:0,2 | Bulk Solution | Aqua bidest (50% des Gesamt wassers) | Hilfsstofflösung lösung (Mannit) | Wässrige Protaminlösung | pH-Wert-Emstellung auf 6,2 | Mit Aqua bidest auf Endgewicht Auffüllen |
| 6 | 1:0,1, 1,0,08 1:0,06, 1:0,04, 1:0,02 | Bulk Solution | Aqua bidest (50%des Gesanu wassers) | Hilfsstofflösung (Mannit) | Wässrige Protaminlösung | pH-Wert-Einstellung auf 6,2 | Mit Aqua bidest auf Endgewich Auffüllen |
| 7 | 1:0,1, 1-0 08 1:0,06, 1:0,041:0,02 | Bulk Solulion | Aqua bidest (50% des Gesamt wassers) | Hilfsstofflösung (Glycin/ Propylengly -kol) | Wässrige Protaminlösung | pH-Wert-Einstellung auf 6,2 | Mit Aqua Mit Aqua bidest auf Endgewicht Auffüllen |
| 8, 9, 10, 11 | 1:0,1,1-0,08 1:0,06,1:0,04 1:0,02 | Bulk Solution | Aqua bidest (50% des Gesamt wassers) | Hilfsstofflösung (Mannit und Glycin/ Propylengly -koI) | Wässrige Protaminlösung | pH-Wert-Einstellung auf 6,2 | Mit Aqua bidest auf Endgewicht auf Endgewicht Auffüllen |
| 12 | 1:0,5; 1:0,2; 1:0,1 1:0,05; 1:0,02 1:0,01 | Bulk Solution | Aqua bidest (50% des Gesamt wassers) | Hilfsstofflösung (Glycin und Glycin/ Propy) | Wässrige Protaminlösung | pH-Wert-Einstellung auf 6,2 | Mit Aqua bidest auf Endgewicht Auffüllen |
| 13 | 0,05 1:0,02 1:0,01 | Bulk Solution | Aqua bidest (50% des Gesamt assers) | Hilfsstofflösung (Mannit und (Mannit und Glycin/ Propylengly | ph-Wert Einstellung auf 7 | Wässrige Protamin Wässrige Protaminlösung | Mit Aqua bidest auf Endgewicht Auffüllen |
| 14 | 1:0,05; 1:0,02 1:0,01 | Bulk Solution | Aqua bidest (50% des Gesamt wassers) | Hilfsstofflösung (Mannit) | Wässrige Protaminlösung | Mit Aqua bidest auf Endgewicht auffüllen | Bestimmung des End pH-Wertes |
| 15 | 1:0,005; 10.001 | Bulk Solution | Aqua bidest (50% des (50% des Gesamt wassers) | Hilfsstofflösung (Mannit) | pH-Wert-Einstellung auf 7 | Wässrige Protaminlösung | Mit Aqua bidest auf Endgewicht auf Endgewicht Auffüllen |
| 16, 17 | 1:267 | Protamin in Mannit und Glycin/ Propylengly koI-Hilfsstofflös ung lösen | Bulk Solution | Bestimmung des pH-Wertes | Senkung des ph-Wertes auf einen End-pH Wert von 6 | | |
| 18, 19 | 1:100; 1:10; 1:1 1:0,1 | Protamin in Mannit und GlycinHilfsstofflös ung (pH = 3,6) lösen | Einstellung des pH-Wertes der Hilfsstofflös -ungen auf einen Wert von 7 | Bulk Solution | Bestimmung der End-ph-Werte | Korrektur des Korrektur der pH-Werte der Endformulienmg -en auf 5,8; 6,0; 6,2; 6,5; | |

Molverhältnisse hGH: Protamin der Versuche 1 bis 19

### Versuch 2

Es handelt sich in diesem Fall um eine wirkstoffhaltige EP2000AQ-Formulierung. Nach Versetzen mit wässriger Protaminlösung hat sich ein weißer flockiger Niederschlag gebildet, der sich nach zirka fünf Minuten am Boden abgesetzt hat.

Herstellungsvorgang der Versuche 3, 4, 5, 6, 7
- 2090 µl Bulk Solution (pH-Wert 7,3) werden vorgelegt
- 505 µl Wasser zugegeben, dies entspricht 50 % des Wassers des gesamten Versuches
- anschließend werden 3040 µl Hilfsstofflösung zudosiert (pH-Wert 3,80)
- an dieser Stelle wird mit 404 µl der wässrigen Protaminlösung versetzt
- bei der erhaltenen Lösung mit 1 M H₃PO₄ ein pH-Wert von 6,2 eingestellt
- Ergänzung mit Aqua bidest auf ein Endvolumen von 6130 µl

### Versuch 3

Aufgrund des Niederschlages der sich unmittelbar nach der Zugabe der Protaminlösung zur Bulk Solution während des Versuchs 2 gebildet hat, ist in diesem Versuch der Herstellungsvorgang der Formulierung insofern abgeändert worden, als die Protaminlösung erst nach Zugabe der Hilfsstofflösung zugesetzt wurde.

Obwohl an der Eintropfstelle der Protaminlösung eine kurzzeitige Trübung zu beobachten war, die wahrscheinlich auf ein konzentrationsbedingtes Grenzflächenphänomen zurückzuführen ist, blieben sowohl die hGH zu Protamin 1:0,02 (0,4 µl Protamin/ml Formulierung) als auch die 1:0,002- Formulierung (0,04 µl Protamin/ml Formulierung) nach langsamen Umrühren klar. Es traten weder eine weitere Trübung noch ein Niederschlag auf.

### Versuch 4

Nach Zudosieren aller Komponenten ist der Ansatz mit molaren Verhältnissen hGH zu Protamin von 1:0,04 (0,8 µg Protamin/ml Formulierung) klar geblieben, es ist kein sichtbarer Niederschlag entstanden.

### Versuch 5

Bei diesen Formulierungen trat bei beiden hGH-Protamin-Verhältnissen schon während der Herstellung an der Eintropfstelle der Protaminlösung eine Trübung auf, die sich durch langsames Rühren nicht wieder beseitigen ließ. Nach einigen Minuten konnten deutlich sichtbare Aggregate beobachtet werden.

### Versuch 6

Auch bei diesem Versuch wurde der Herstellungsvorgang von früheren Versuchen übernommen und jede der eingesetzten Komponenten vor dem Zudosieren sterilfiltriert. Bei allen Verdünnungen konnte eine leichte Trübung beobachtet werden, wobei der hGH-Protamin-Ansatz mit einem molaren Verhältnis 1:0,1 (2 µg Protamin/ml Formulierung) stärker getrübt ist, als jener der ein Molverhältnis von 1:0,02 (0,4 µg Protamin/ml Formulierung) aufweist.

### Versuch 7

Bei diesem Versuch wurde wie bei Versuch 6 der Zuckeralkohol Mannit durch die Stabilisatoren Glycin und Propylenglykol ersetzt. Die molaren Verhältnisse zwischen hGH und Protamin reichten von 1:0,1 (2 µg Protamin/ml Formulierung) über 1:0,08 (1,6 µg Protamin/ml Formulierung) 1:0,06 (1,2 µg Protamin/ml Formulierung), 1:0,04 (0,8 µg

Protamin/ml Formulierung) bis 1:0,02 (0,4 µg Protamin/ml Formulierung). Bei allen Verdünnungen kam es nach Zugabe der Hilfsstofflösung zu einer Schlierenbildung die sich nach langsamen Umschütteln wieder aufgelöst hat. Weder die 1:0,1 noch die 1:0,08 Verdünnung zeigten nach Zugabe der Protaminlösung eine Trübung auf. Aus diesem Ergebnis konnte abgeleitet werden, dass Glycin als amphiphiles Molekül wahrscheinlich mit beiden Proteinen interagiert und somit eine Aggregatbildung verhindern werden kann. Die Endformulierungen dieses Versuches waren alle klar gelöst.

### Herstellungsvorgang der Versuche 8, 9, 10, 11

In Anlehnung an die bisherige Formulierungsarbeit war die nächste Überlegung, inwieweit jeder einzelne Hilfsstoff Einfluss auf mögliche Interaktionen zwischen hGH und Protamin hat. Zu diesem Zweck wurden parallel Mannit- und Glycin-Propylenglykol-Formulierungen hergestellt, wobei pro Versuch jeweils eine Komponente der Hilfsstofflösung entfernt wurde.

Angefangen von Benzylalkohol über Poloxamer 188, Propylenglykol und Mannit wurde kontinuierlich die Anzahl der Komponenten reduziert. Versuch 11 bestand aus Pufferlösung und Glycin bzw. der reinen Pufferlösung. Unmittelbar nach der Herstellung wurden die Endformulierungen mittels pH-Meter vermessen.

### Versuche 8, 9, 10

Bei diesen Versuchen die zum einen Mannit, zum anderen Glycin und Propylenglykol enthalten, wurden wiederum molare Verhältnisse von hGH zu Protamin im Bereich von 1:0,1 über 1:0,08, 1:0,06, 1:0,04 bis 1:0,02 hergestellt.

Versuch 8: Es waren alle Komponenten der Formulierungen enthalten: Nach Zugabe der Hilfsstofflösung war bei jeder Verdünnung eine vorübergehende Schlierenbildung zu beobachten. Wahrscheinlich wurde der IP von hGH durchschritten. Sowohl bei einem Molverhältnis von 1:0,1 als auch bei jenem von 1:0,08 war in der Endformulierung keine Trübung zu beobachten. Es muss jedoch damit gerechnet werden, dass aufgrund der kleinen Ansatzmenge ein Teil des Protamins am Sterilfilter adsorbiert wurde, und somit nicht die gewünschte Protaminkonzentration in der Endformulierung vorhanden war. Daher wurde in späteren Versuchen die Menge der Protaminlösung erhöht und der Sterilfilter zuvor mit Protamin abgesättigt.

Versuch 9: Bei diesen beiden Formulierungen wurde bei der Herstellung jeweils auf den Zusatz des Konservierungsmittels Benzylalkohol verzichtet. Die molaren Verhältnisse wurden beibehalten, wobei wiederum weder bei einem Molverhältnis von 1:0,1 noch bei einem von 1:0,08 eine Trübung der Endformulierung zu beobachten war. Es muss auch in diesem Fall damit gerechnet werden, dass der Versuch nicht die gewünschte Protaminkonzentration enthält.

Für die Endformulierungen konnten folgende pH-Werte ermittelt werden:

| Molverhältnis hGH: Protamin | Aussehen Mannit-Formulierung | pH-Wert Mannit-Formulierung | Aussehen Gly/PG-Formulierung | pH-Wert GIy/PG Formulierung |
|---|---|---|---|---|
| 1:0,1 | klare Lösung | 6,14 | klare Lösung | 4,16 |
| 1:0,08 | klare Lösung | 5,82 | klare Lösung | 4,10 |
| 1:0,06 | klare Lösung | 6,33 | klare Lösung | 4,25 |
| 1:0,04 | klare Lösung | 4,88 | klare Lösung | 4,14 |
| 1:0,02 | klare Lösung | 6.0 | klare Lösun | 4,12 |

### Versuch 9: pH-Wert der Endformulierungen

Versuch 10: Die bei diesem Versuch verwendeten Rezepturen enthalten weder Benzylalkohol noch Propylenglykol. Auch hier konnte eine kurzzeitige Schlierenbildung beobachtet werden. Die Endformulierungen sind alle klar gelöst und die Protaminkonzentration aufgrund von Adsorbtionsphänomenen wahrscheinlich geringer als beabsichtigt.

Die nachstehende Tabelle zeigt den pH-Wert der Endformulierungen:

| Molverhältnis hGH: Protamin | Aussehen Mannit-Formulierung | pH-Wert Mannit-Formulierung | Aussehen Gly/PG-Formulierung | pH-Wert GIy/PG Formulierung |
|---|---|---|---|---|
| 1:0,1 | klare Lösung | 6,02 | klare Lösung | 4,27 |
| 1: 0,08 | klare Lösung | 6,12 | klare Lösung | 4,07 |
| 1: 0,06 | klare Lösung | 6,23 | klare Lösung | 4,15 |
| 1:0,04 | klare Lösung | 6,33 | klare Lösung | 4,11 |
| 1:0,02 | klare Lösung | 6,23 | klare Lösung | 4,16 |

### Versuch 10: pH-Wert der Endformulierungen

### Versuch 11

Um den Filter vor der Sterilfiltration mit Protamin abzusättigen, wurde für die Protaminstammlösung ein größerer Ansatz berechnet. Bei den Lösungen die Glycin und Propylenglykol enthalten, konnte nach Zugabe der Hilfsstofflösung eine kurzzeitige Trübung beobachtet werden, die wieder verschwand. Die Endformulierungen des Glycin/Propylenglykol-Ansatzes sind alle klar gelöst, beim Mannit-Ansatz war ab einem molaren Verhältnis hGH zu Protamin von 1:0,06 (1,2 µg Protamin/ml Formulierung) mit steigender Protaminkonzentration eine Trübung zu sehen.

Es wird davon ausgegangen, dass bei diesem Versuch die gewünschten Protaminmengen in den Proben enthalten sind, und es dadurch ab einer bestimmten Protaminkonzentration wieder zu einer Trübung der mannithaltigen Lösungen gekommen ist.

Die Vermessung der Endformulierungen im pH-Meter lieferte folgendes Ergebnis:

| Molverhältnis hGH:Protamin | Aussehen Mannit-Formulierung | pH-Wert Mannit-Formulierung | Aussehen Gly/PG Formulierung | pH-Wert Gly/PG-Formulierung |
|---|---|---|---|---|
| 1:0,1 | Trübung | 6,32 | klare Lösung | 4,26 |
| 1:0,08 | Trübung | 6,45 | klare Lösung | 4,28 |
| 1:0,06 | leicht opaleszierend | 6,44 | klare Lösung | 4,27 |
| 1:0,04 | klar gelöst | 6,36 | klare Lösung | 4,24 |
| 1:0,02 | klar gelöst | 6,34 | klare Lösung | 4,32 |

### Versuch 11: pH-Wert der Formulierungen

Herstellungsvorgang der Versuche 12, 13, 14, 15
- 2090 µl Bulk Solution (pH-Wert 7,3) werden vorgelegt
- 1010 µl Wasser werden zugegeben, dies entspricht 50 % des Wassers des gesamten Versuches
- anschließend werden 6080 µl Hilfsstofflösung zudosiert (pH-Wert 3,80)
- an dieser Stelle wird mit 808 µl der wässrigen Protaminlösung versetzt
- bei der so erhaltenen Lösung wird mit 1 M H₃PO₄ bzw. 0,1 M H₃PO₄ ein pH-Wert von 6,2 eingestellt
- Ergänzung mit Aqua bidest auf ein Endvolumen von 6130 µl

### Versuch 12

Wie bereits erwähnt, wurde ab diesem Zeitpunkt das Hauptaugenmerk auf die Glycin/Propylenglykol bzw. Glycin-Formulierung gelegt, da mannithaltige Lösungen eher zu getrübten Lösungen neigten. hGH und Protamin wurden in den Molverhältnissen 1:0,5 (0,10 µg Protamin/ml Formulierung); 1:0,2 (0,04 µg Protamin/ml Formulierung); 1:0,1 (0,02 µg Protamin/ml Formulierung); 1:0,05 (0,01 µg Protamin/ml Formulierung); 1:0,02 (0,004 µg Protamin/ml Formulierung) und 1:0,01 (0,002 µg Protamin/ml Formulierung) eingesetzt. Nach Zugabe der Glycin- bzw. Glycin/Propylenglykol-Hilfsstofflösung kam es bei allen Lösungen zu einer kurzzeitigen Trübung, die nach zudosieren weiterer Hilfsstofflösung wieder verschwand. Diese kurzzeitige Trübung könnte mit dem Durchschreiten des IP erklärt werden. Sowohl jene Formulierungen die Glycin und Propylenglykol enthalten, als auch jene ohne Propylenglykol waren klare Lösungen von denen folgende pH-Werte bestimmt wurden:

| Molverhältnis hGH: Protamin | Aussehen Glycin Formulierung | pH-Wert Glycin Formulierung | Aussehen Gly/PG Formulierung | pH-Wert GIy/PG Formulierung |
|---|---|---|---|---|
| 1:0,5 | klare Lösung | 3,92 | klare Lösung | 4,40 |
| 1: 0,2 | klare Lösung | 3,91 | klare Lösung | 4,42 |
| 1: 0,1 | klare Lösung | 3,93 | klare Lösung | 4,39 |
| 1:0,05 | klare Lösung | 3,93 | klare Lösung | 4,38 |
| 1: 0,02 | klare Lösung | 3,97 | klare Lösung | 4,36 |
| 1: 0,01 | klare Lösung | 3.90 | klare Lösung | 4,50 |

### Versuch 12: pH-Wert der Formulierungen

Bei beiden Formulierungen (Glycin/Propylenglykol und Glycin) konnte nur bei den molaren Verhältnissen 1:0,02 und 1:0,01 ein pH-Wert von 5,8 eingestellt werden, ohne dass eine Trübung zu beobachten war. Bei allen anderen geprüften hGH-Protamin-Formulierungen kam es ab einem pH-Wert von 5,8 zu einer Trübung. Nach der Korrektur des pH-Wertes auf einen Wert von 6,2 waren alle Lösungen die Glycin und Propylenglykol enthalten getrübt, wogegen jene mit Glycin bei den niedrigen Verdünnungen (Molverhältnisse 1:0,05; 1:0,02; und 1:0,01) klar geblieben sind.

### Versuch 13

Es wurden vergleichsweise Untersuchungen mit der Mannit-Formulierung vorgenommen. Der Herstellungsvorgang wurde beibehalten, die Versuchgröße verdoppelt und vor dem zudosieren der wässrigen Protaminlösung mit 0,5 M Natronlauge ein pH-Wert von 7 eingestellt. Damit sollte ein Durchschreiten des IP beim späteren Alkalisieren verhindert werden.

Die pH-Wert-Messungen der Mischungen aus Bulk Solution, Wasser und Hilfsstofflösung ergaben:

| pH-Wert Formulierung (Gly/PG) | pH-Wert Formulierung (Mannit) |
|---|---|
| 4,05 | 6,99 |
| 4,20 | 6,18 |
| 4,05 | 6,22 |

### Versuch 13: pH-Werte der Mischung aus Bulk Solution, Wasser und Hilfsstofflösung

Im Anschluss wurde die wässrige Protaminlösung hinzugefügt und mit Wasser zum Schlussgewicht aufgefüllt. Für die Endformulierungen ermittelte das pH-Meter folgende Werte:

| Molverhältnis hGH: Protamin | Aussehen GlyI/PG-Formulierung | pH-Wert Gly/PG Formulierung | Aussehen Mannit-Formulierung | pH-Wert Mannit-Formulierung |
|---|---|---|---|---|
| 1:0,05 | klare Lösung | 7,15 | schwach opaleszierend | 7,15 |
| 1:0,02 | klare Lösung | 7,01 | schwach opaleszierend | 7,11 |
| 1:0,01 | klare Lösung | 7,41 | klare Lösung | 7,14 |

### Versuch 13: pH-Werte der Formulierung

Nach Prüfung des pH-Wertes wurde mit Phosphorsäure ein Ziel-pH-Wert von 6,2 eingestellt. Der Glycin/Propylenglykol-Ansatz war klar gelöst, der Mannit-Ansatz opaleszierend.

Nach pH-Wert-Korrektur und Abfüllung in 2R-Vials wurden die Ansätze bei folgenden Werten gelagert:

| Molverhältnis hGH: Protamin | Aussehen Gly/PG-Formulierung | pH-Wert Gly/PG Formulierung | Aussehen Mannit-Formulierung | pH-Wert Mannit-Formulierung |
|---|---|---|---|---|
| 1:0,05 | schwach opaleszierend | 6,33 | schwach opaleszierend | 6,12 |
| 1:0,02 | schwach opaleszierend | 6,5 | schwach opaleszierend | 6,4 |
| 1:0,01 | klare Lösung | 6,42 | klare Lösung | 6,13 |

### Versuch 13: pH-Werte der Endformulierung nach pH-Wert-Korrektur

Es wird angenommen, dass bei einem molaren Verhältnis von 1:0,01 nicht der Einfluss bestimmter Hilfsstoffe, sondern die sehr geringe Protaminkonzentration den Grund für die Klarheit der Lösungen darstellt. Dieser Versuch hat gezeigt, dass die Senkung des pH-Wertes der Endformulierungen unter einen Wert von 6,5 bei einem Teil der Präparate zu einer Trübung führt und diese von der Protaminmenge abhängig ist.

### Versuch 14

Bei diesem Versuch hat man auf die pH-Wert-Korrektur während des Herstellungsprozesses verzichtet. Es wurde lediglich der pH-Wert der Endformulierungen ermittelt und geringfügig angepasst. Es kam hier nur eine Mannit-Hilfsstofflösung zum Einsatz, hGH und Protamin wurden in molaren Verhältnissen von 1:0,05 (1 µg Protamin/ml Formulierung); 1:0,02 (0,4 µg Protamin/ml Formulierung); sowie 1:0,01 (0,2 µg Protamin/ml Formulierung) zusammengefügt.

| Molverhältnis hGH: Protamin | Aussehen Mannit-Formulierung | pH-Wert Mannit-Formulierung |
|---|---|---|
| 1:0,05 | schwach opaleszierend | 6,18 |
| 1:0,02 | schwach opaleszierend | 6,17 |
| 1: 001 | sehr schwach opaleszierend | 6,25 |

### Versuch 14: pH-Werte der Formulierung

Der pH-Wert und das Aussehen jener Formulierungen die nach pH-Wert-Korrektur mit 1M Phosphorsäure auf Stabilität gelegt wurden sind aus folgender Tabelle ersichtlich:

| Molverliältnis hGH: Protamin | Aussehen Mannit-Formulierung | pH-Wert Mannit-Formulierung |
|---|---|---|
| 1:0,05 | schwach opaleszierend | 6,18 |
| 1:0,02 | schwach opaleszierend | 6,10 |
| 1:0,01 | sehr schwach opaleszierend | 6,08 |

### Versuch 14: pH-Werte der Endformulierung nach pH-Wert-Korrektur

Jede der Verdünnungen ist opaleszierend, der pH-Wert konnte nicht unter einen Wert von 6 gesenkt werden, ohne dass eine Trübung sichtbar war.

### Versuch 15

Bei diesem Versuch wurde die Protaminkonzentration im Vergleich zu früheren Versuchen wesentlich verringert. hGH und Protamin wurden in molaren Verhältnissen von 1:0,005 (0,1 µg Protamin/ml Formulierung) und 1:0,001 (0,02 µg Protamin/ml Formulierung) eingesetzt. Es wurde sowohl die Mannit- als auch die Glycin/Propylenglykol-Rezeptur verwendet. Wiederum wurden die pH-Werte der Mischungen aus Bulk Solution, Wasser und Hilfsstofflösung ermittelt, es ergaben sich folgende Werte:

| Molverhältnis hGH: Protamin | pH-Wert Mannit-Formulierung | pH-Wert Gly/PG-Formulierung |
|---|---|---|
| 1:0,005 | 6,09 | 3,57 |
| 1: 0,001 | 6,00 | 3,68 |

### Versuch 15: pH-Werte der Mischung aus Bulk Solution, Wasser und Hilfsstofflösung

Nach Korrektur des pH-Wertes auf 7, hinzufügen der Protaminlösung sowie Auffüllen mit Wasser auf das Zielvolumen, konnten für die Endformulierungen folgende Werte bestimmt werden.

| **Molverhältnis hGH: Protamin** | **Aussehen Gly/PG-Formulierung** | **pH-Wert Gly/PG-Formulierung** | **Aussehen Mannit-Formulierung** | **pH-Wert Mannit-Formulierung** |
|---|---|---|---|---|
| **1:0,005** | klare Lösung | 7,05 | klare Lösung | 7,20 |
| **1: 0,001** | klare Lösung | 7,02 | klare Lösung | 7,06 |

### Versuch 15: pH-Werte der Formulierung

Keine der Verdünnungen wies eine Trübung auf. Nach pH-Wert- Korrektur ergaben sich für die Endformulierungen folgende Werte, wobei alle Lösungen klar sind.

| **Molverhältnis hGH: Protamin** | **Aussehen Gly/PG-Formulierung** | **pH-Wert Gly/PG-Formulierung** | **Aussehen Mannit-Formulierung** | **pH-Wert Mannit-Formulierung** |
|---|---|---|---|---|
| **1: 0,006** | klare Lösung | 5,1 | klare Lösung | 5,9 |
| **1:0,001** | klare Lösung | 5,7 | klare Lösung | 5,8 |

### Versuch 15: pH-Werte der Endformulierung nach pH-Wert-Korrektur

Bei diesem Versuch ist es wahrscheinlich auf die geringe Protaminkonzentration zurückzuführen, dass trotz eines End-pH-Wertes von 5 keine Trübung aufgetreten ist.

Herstellungsvorgang der Versuche 16, 17, 18, 19
- pH-Wert-Korrektur der Hilfsstofflösung auf einen geforderten Wert
- Einführen von Protamin als zusätzliche Komponente der Hilfsstofflösungen; Protamin wird direkt in der Hilfsstofflösung gelöst
- Verdünnung der Stamm-Hilfsstofflösung mit weiterer Hilfsstofflösung um die gewünschte Protaminkonzentration zu erreichen
- Vorsichtiges Zudosieren der Bulk Solution
- Einstellung des Ziel-pH-Wertes der Endformulierung
- Abzentrifugieren eventuell auftretender Niederschläge
- Abfüllung in 2R-Vials und Lagerung bei Raumtemperatur bzw. bei 2-8° C

### Versuch 16

Der Herstellungsvorgang wurde insofern abgeändert, als man den Hilfsstoff Protamin nicht als wässrige Lösung zugesetzt, sondern direkt in der Hilfsstofflösung, die auf einen pH-Wert von 7 eingestellt wurde, gelöst hat. Die Bulk Solution wurde in 30 µl-Schritten unter ständigem Umschütteln zur Hilfsstofflösung, die jetzt das Protamin (75 mg Protamin/ml Formulierung) in großem Überschuss enthält, zudosiert.

Die Ansätze zeigten keine Trübungen auf, mittels pH-Meter wurden folgende Werte ermittelt.

| **Molverhältnls hGH: Protamin** | **Aussehen Gly/PG-Formulierung** | **pH-Wert Gly/PG-Formulierung** | **Aussehen Mannit-Formulierung** | **pH-Wert Mannit Formulierung** |
|---|---|---|---|---|
| **1:267** | klare Lösung | 12,09 | klare Lösung | 10,96 |

### Versuch 16: pH-Werte der Formulierung

Im Anschluss wurde der pH-Wert mit 1M Phosphorsäure auf einen Ziel-pH-Wert von 6,0 gesenkt, die Formulierungen sind klar geblieben.

### Versuch 17

Die Herstellung des Versuches erfolgte analog zu Versuch 16, mit dem Unterschied, dass bei diesem Versuch die Hilfsstofflösung anstatt eines pH-Wertes von 7 einen von 3,6 aufwies.

| **Molverhältnis hGH: Protamin** | **Aussehen Gly/PG-Formulierung** | **pH-Wert Gly/PG-Formulierung** | **Aussehen Mannit-Formulierung** | **pH-Wert Mannit Formulierung** |
|---|---|---|---|---|
| 1: 267 | klare Lösung | 12,70 | klare Lösung | 12,60 |

### Versuch 17: pH-Werte der Formulierung

Es wurde wiederum mit 1M Phosphorsäure auf einen pH-Wert von 6 korrigiert, wobei bei keiner der Formulierungen eine Trübung zu beobachten war.

Die Versuche 16 und 17 zeigten auf, dass es bei einem Überschuss von Protamin in einem Molverhältnis von 1:267 (75 mg Protamin/ml Formulierung) unabhängig vom voreingestellten pH-Wert und der Zusammensetzung der Hilfsstofflösung, zu keiner Trübung der Formulierungen kommt.

### Versuche 18, 19

hGH und Protamin wurden für beide Rezepturen in den Molverhältnissen 1:100 (53,5 mg Protamin/ml Formulierung); 1:10 (5,35 mg Protamin/ml Formulierung); 1:1 (0,53 mg Protamin/ml Formulierung); 1:0,1 (0,05 mg Protamin/ml Formulierung) eingesetzt. Die vier Endformulierungen wurden schlussendlich auf die pH-Werte 6,5; 6,2; 6,0 und 5,8 korrigiert. Bei den Hilfsstofflösungen wurde nach Protaminzugabe der pH-Wert auf 7 eingestellt. Vor dieser Korrektur wurden folgende Werte gemessen:

| **Molverhältnis hGH: Protamin** | **pH-Wert Glycin-Hilfsstofflösung** | **pH-Wert Mannit- Hilfsstofflösung** |
|---|---|---|
| **1:100** | 12,44 | 12,69 |
| **1:10** | 9,23 | 11,25 |
| **1:1** | 7,64 | 6,87 |
| **1: 0.1** | 6,56 | 6,38 |

### Versuche 18,19: pH-Wert der Hilfsstofflösungen

Bei den vermessenen Lösungen wurde im Anschluss ein pH-Wert von 7 eingestellt. Nach Zugabe der Bulk Solution wurden folgende Werte ermittelt:

| **Molverhältnis hGH: Protamin** | **Aussehen Glycin-Formulierung** | **pH-Wert Glycin-Formulierung** | **Aussehen Mannit-Formulierung** | **pH-Wert Mannit Formulierung** |
|---|---|---|---|---|
| **1:100** | klare Lösung | 7,04 | klare Lösung | 7,05 |
| **1:10** | Leicht opaleszierend | 7,14 | leicht opaleszierend | 7,12 |
| **1:1** | starke Trübung | 7,12 | starke Trübung | 7,11 |
| **1:0,1** | schwache Trübung | 7,00 | schwache Trübung | 7,05 |

### Versuche 18,19: pH-Werte der Formulierung

Die pH-Wert- Korrektur der Versuche 18 und 19 erfolgte mit 1M Phosphorsäure bzw. 0,1 M Phosphorsäure.

Das Aussehen der Lösungen ist in der nachstehenden Tabelle zusammengefasst.

| **Molverhältnis hGH: Protamin** | **Aussehen Glycin-Formulierung** | **pH-Wert Glycin-Formulierung** | **Aussehen Mannit-Formulierung** | **pH-Wert Mannit Formulierung** |
|---|---|---|---|---|
| **1:100** | klare Lösung | 6,5 | klare Lösung | 6,5 |
| **1:100** | klare Lösung | 6,2 | klare Lösung | 6,2 |
| **1:100** | klare Lösung | 6,0 | klare Lösung | 6,0 |
| **1:100** | klare Lösung | 5,8 | klare Lösung | 5,8 |
| **1:10** | schwach opaleszierend | 6,5 | schwach opaleszierend | 6,5 |
| **1:10** | schwach opaleszierend | 6,2 | schwach opaleszierend | 6,2 |
| **1:10** | schwach opaleszierend | 6,0 | schwach opaleszierend | 6,0 |
| **1:10** | schwach opaleszierend | 5,8 | schwach opaleszierend | 5,8 |
| **1:1** | schwache Trübung | 6,5 | starke Trübung | 6,5 |
| **1:1** | starke Trübung | 6,2 | starke Trübung | 6,2 |
| **1:1** | starke Trübung | 6,0 | starke Trübung | 6,0 |
| **1:1** | starke Trübung | 5,8 | starke Trübung | 5,8 |
| **1:0,1** | schwache Trübung | 6,5 | schwache Trübung | 6,5 |
| **1:0,1** | schwache Trübung | 6,2 | schwache Trübung | 6,2 |
| **1:0,1** | schwache Trübung | 6,0 | schwache Trübung | 6,0 |
| **1:0,1** | starke Trübung | 5,8 | schwache Trübung | 5,8 |

### Versuche 18, 19: Aussehen und pH-Werte der eingestellten Endformulierungen

Die Ergebnisse der Versuche 18 und 19 machen deutlich, dass es lediglich bei einem molaren hGH-Protamin-Verhältnis von größer als 1:10 möglich ist, klare Lösungen zu erhalten. Jedoch hat die Wahl der Hilfsstofflösung hier wenig Einfluss, da für die Mannit und die Glycin-Formulierung die gleichen Ergebnisse erzielt wurden.

In weiteren Versuchen wurde als das minimale Molverhältnis von Somatropin zu Protamin zu 1:13 ermittelt.

Die in der vorangehenden Beschreibung, den Ansprüchen und den Zeichnungen offenbarten Merkmale der Erfindung können sowohl einzeln als auch in beliebiger Kombination zur Verwirklichung der Erfindung in ihren verschiedenen Ausführungsformen wesentlich sein.

## Patentansprüche

1. Flüssigformulierung umfassend einen Wirkstoff und Protamin, wobei die Flüssigformulieung verschieden ist von einer Suspension.

2. Flüssigformulierung nach Anspruch 1, **dadurch gekennzeichnet, dass** die Flüssigformulierung eine klare Flüssigformulierung ist.

3. Flüssigformulierung nach einem der Ansprüche 1 bis 2, **dadurch gekennzeichnet, dass** die Flüssigformulierung eine Trübung aufweist, die nicht stärker ist als die Trübung der Referenzlösung IV wie definiert in Ph.Eu 1997, 2.2.1.

4. Flüssigformulierung nach einem der Ansprüche 1 bis 3, **dadurch gekennzeichnet, dass** das Protamin Lachsspermaprotamin ist.

5. Flüssigformulierung nach einem der Ansprüche 1 bis 4, **dadurch gekennzeichnet, dass** das Molverhältnis von Wirkstoff zu Protamin mindestens 1:2 oder mehr beträgt, bevorzugterweise mindestens 1:10 und bevorzugtererweise mindestens 1: 100.

6. Flüssigformulierung nach einem der Ansprüche 1 bis 5, **dadurch gekennzeichnet, dass** das Molverhältnis von Wirkstoff zu Protamin mindestens 1: 13 beträgt.

7. Flüssigformulierung nach einem der Ansprüche 1 bis 6, **dadurch gekennzeichnet, dass** der Wirkstoff ausgewählt ist aus der Gruppe umfassend Peptide, Polypeptide und Proteine.

8. Flüssigformulierung nach einem der Ansprüche 1 bis 7, **dadurch gekennzeichnet, dass** der Wirkstoff ein Molekulargewicht von etwa 3.000 Da bis etwa 200.000 Da, bevorzugterweise etwa 10.000 bis etwa 100.000 Da aufweist.

9. Flüssigformulierung nach einem der Ansprüche 1 bis 8, **dadurch gekennzeichnet, dass** der Wirkstoff ausgewählt ist aus der Gruppe, die Wachstumsfaktoren, Cytokine, Interferone, Interleukine und Antikörper umfasst.

10. Flüssigformulierung nach einem der Ansprüche 1 bis 9, **dadurch gekennzeichnet, dass** der Wirkstoff hGH ist.

11. Flüssigformulierung nach einem der Ansprüche 1 bis 10, **dadurch gekennzeichnet, dass** die Flüssigformulierung einen pH-Wert von etwa 4 bis 10, bevorzugterweise von etwa 5,5 bis 7 aufweist.

12. Flüssigformulierung nach einem der Ansprüche 1 bis 11, **dadurch gekennzeichnet, dass** die Flüssigformulierung mindestens einen weiteren Hilfsstoff enthält, wobei der Hilfsstoff ausgewählt ist aus der Gruppe umfassend Glycin, Propylenglykol, Mannit und Poloxamer 188.

13. Flüssigformulierung nach einem der Ansprüche 1 bis 13, zur Verwendung in einem Verfahren zur Behandlung einer Erkrankung.

14. Verwendung von Protamin zur Herstellung einer Flüssigformulierung.

15. Verwendung von Protamin als Lösungsvermittler.

16. Verwendung nach Anspruch 15, wobei die Verwendung im Rahmen der Herstellung einer Flüssigformulierung erfolgt.

17. Verfahren zur Herstellung einer einen Wirkstoff und Protamin umfassenden Flüssigformulierung umfassend die Schritte:
a) Bereitstellen von Protamin;
b) Bereitstellen des Wirkstoffes; und
c) Formulieren von Protamin und des Wirkstoffes zu einer Flüssigformulierung.
